# EUROPEAN PATENT APPLICATION

(11) **EP 4 847 082 A1**
(43) Date of publication of application: **02.12.2026**
(21) Application number: 24919595.9
(22) Date of filing: 10.05.2024
(51) Int. Cl.: A61N 5/06, A61B 3/11, G02B 27/09

(54) **MYOPIA ILLUMINATION SYSTEM, MYOPIA ILLUMINATION APPARATUS, AND CONTROL METHOD THEREOF**

(30) Priority: 26.01.2024 CN 202420201949 U; 16.04.2024 CN 202410456292
(71) Applicant: Beijing Airdoc Technology Co., Ltd., Beijing 100089 (CN); Shanghai Airdoc Medical Technology Co., Ltd., Shanghai 200233 (CN); Changsha Airdoc Health Technology Co., Ltd., Changsha, Hunan 410000 (CN)
(72) Inventor: ZHAO, Zhifei, Beijing 100089 (CN); HE, Chao, Beijing 100089 (CN); CHANG, Xiangang, Beijing 100089 (CN); LI, Ruixia, Beijing 100089 (CN); WU, Xiaolei, Beijing 100089 (CN); ZHANG, Dalei, Beijing 100089 (CN)
(74) Representative: advotec.
(86) International application number: PCT/CN2024/092324
(87) International publication number: WO 2025/156506

(57) **Abstract**

Provided are a myopia illumination system (100), a myopia illumination device (1400), and a control method (1600) thereof. The myopia illumination system (100) comprises: a broad-spectrum light source (1) configured for emitting a broad-spectrum light; and a projection system (3) configured for projecting the broad-spectrum light to the eyes of a user. The broad-spectrum light source (1) can improve irradiation safety and avoid the risk of high-power stimulation of the fundus.

## Description

### CROSS-REFERENCE TO RELATED APPLICATIONS

This disclosure claims the benefit of priority to Chinese patent application No. 202420201949.7, filed on January 26, 2024 and titled "a myopia illumination system", and Chinese patent application No. 202410456292.3, filed on April 16, 2024 and titled "a myopia illumination system, a myopia illumination device and a control method for a myopia illumination device".

### TECHNICAL FIELD

The disclosure generally relates to the technical field of myopia prevention and control devices; and in particular to a myopia illumination system, a myopia illumination device and a control method for a myopia illumination device.

### BACKGROUND

Axial length growth is one of the main factors causing myopia in the human eye, especially for adolescents in a rapid growth period. Studies have shown that directly irradiating the retina with 650 nm long-wave red light may effectively suppress the growth of the axial length. In addition, studies have also shown that irradiating the retina of the fundus via a light source of an appropriate power with a specific wavelength for an appropriate duration may promote blood flow in the choroid beneath the retina, improve the hypoxia state of the choroid, so that the choroid thickens, achieving an effect of myopia prevention and control and improvement. Thus, irradiating the retina with the light source in the specific wavelength has a positive impact on myopia prevention and control for adolescents.

Existing light feeding instruments (i.e., Repeated Low-Level Red-Light (RLRL) therapy devices) usually adopt a red-light laser diode (LD) as a light source. The LD has characteristics of monochromaticity, coherence, directionality and collimation. The characteristics cause a risk of stimulating the fundus with a high power during use of the light feeding instruments. In addition, in the case of using the LD, there is a phenomenon of wavelength shift, which causes that the light of the expected wavelength is unable to irradiate the fundus. Further, when the human eye directly looks at the light source or observes a fundus spot after lens transformation, the human eye may adjust the fovea to face the light source. Thus, the spot energy is concentrated at the macular fovea, which is the most light-sensitive position, causing strong stimulation to the eyes of the user and even retinal damage.

In view of the above situation, it is desired to provide a novel myopia illumination system, which may effectively control the light and/or spot desired to be irradiated to the fundus, thus avoiding the risk of stimulating the fundus with the high power. Further, it is also desired to effectively control that the wavelength of the light desired may be irradiated to the fundus.

### SUMMARY

In order to solve at least one or more of the technical problems mentioned above, the disclosure provides a myopia illumination system.

In a first aspect, the disclosure provides a myopia illumination system. The myopia illumination system includes: a broad-spectrum light source, configured to emit broad-spectrum light; and a projection system, configured to project the broad-spectrum light onto eyes of a user.

In some embodiments, the myopia illumination system further includes: a beam shaping system, configured to shape the broad-spectrum light to form annular light; and the projection system is configured to project the annular light onto the eyes of the user to form an annular spot.

In some embodiments, the beam shaping system includes: a substrate, the substrate including an annular transparent portion and an opaque portion located at a center of the annular transparent portion; or the beam shaping system includes: a spectrum shaper, the spectrum shaper including an annular transparent portion and an opaque portion located at a center of the annular transparent portion.

In some embodiments, the myopia illumination system further includes: a visual target system, configured to generate light of a visual target pattern, in which the light generated is projected onto the eyes of the user together with the annular light.

n some embodiments, the visual target system includes: a visual target transparent portion arranged in a spectrum shaper of the beam shaping system and located at a center of an opaque portion of the spectrum shaper, and the visual target transparent portion is configured to form the annular light with the visual target pattern at the center by transmitting a portion of the broad-spectrum light.

In some embodiments, the visual target system includes: a visual target light source and a beam splitter, an output optical path of the visual target light source coinciding with a first optical path of the beam splitter, and a second optical path of the beam splitter coinciding with a propagation optical path of the annular light; in which the first optical path is one of a reflection optical path or a projection optical path, the second optical path is the other of the reflection optical path or the projection optical path different from the first optical path, and a position where a visual target light output by the visual target light source is projected onto the eyes of the user is located at a center of the annular spot.

In some embodiments, the myopia illumination system includes at least one of the following features (1) to (3): (1) a material of the annular transparent portion includes any one of: glass, crystal, plastic, or resin; (2) a material of the opaque portion is an opaque material; or (3) the opaque portion is made of the same material as the annular transparent portion but is subjected to an opaque treatment.

In some embodiments, the opaque portion includes: black acrylic; or the opaque treatment includes: sandblasting, painting, or attaching a black-dot annular transparent film.

In some embodiments, a size of the annular spot satisfies any one of following situations: an annular width k of the annular spot is 0.0038 mm to 3 mm, and a distance r1 from a spot center to an annular centerline is 0.0038 mm to 3 mm; an inner diameter of the annular spot is between 0.0076 mm and 6 mm; an inner diameter d1 of the annular spot is 1.5 mm, and an outer diameter d2 of the annular spot is 2.5 mm; the inner diameter d1 of the annular spot is 2.5 mm, and the outer diameter d2 of the annular spot is 5.5 mm; or the inner diameter d1 of the annular spot is 1.5 mm, and the outer diameter d2 of the annular spot is 5.5 mm.

In some embodiments, a spectral range of the broad-spectrum light source is a spectral range covering any spectral cutoff width within a range of 300 nm to 700 nm.

In some embodiments, the broad-spectrum light source includes at least one of a light-emitting diode (LED) lamp, an incandescent lamp, or a fluorescent lamp.

In some embodiments, the myopia illumination system further includes a spectrum shaping system, configured to form light with a specified spectrum by shaping a spectrum of the broad-spectrum light; and the projection system is configured to project the light with the specified spectrum onto the eyes of the user.

In some embodiments, the spectrum shaping system includes one or more spectrum shapers, and a spectral range of one or more of the spectrum shapers satisfies any one of following situations: a spectral cutoff width is 5 nm to 60 nm, and a central wavelength is selected from any wavelength within a wavelength range of 360 nm to 400 nm or within a wavelength range of 640 nm to 700 nm; a low-pass cutoff range is approximately 640 nm to 660 nm, and a high-pass cutoff range is approximately 670 nm to 700 nm; or a low-pass cutoff range is approximately 360 nm to 375 nm, and a high-pass cutoff range is approximately 385 nm to 400 nm.

In some embodiments, the spectrum shaper includes any one or more or a combination of: a filter; a transmission grating filter; a reflection grating filter; a prism; a lens; a liquid crystal spectrum transmission plate; or a spectrum absorber.

In some embodiments, a spectral characteristic of the spectrum shaper includes any one of: high-pass; low-pass; band-pass; or multi-band-pass.

In some embodiments, the myopia illumination system further includes: a color temperature adjuster, configured to adjust a color temperature of light to be projected onto fundus of the user by transmitting light with a specified color temperature range. Preferably, the color temperature adjuster is a warm-light transparent plate, and the specified color temperature range is approximately 3500 K to 5000 K.

In some embodiments, the myopia illumination system further includes a light homogenizing plate, in which a smooth surface of the light homogenizing plate faces the broad-spectrum light source, and a rough surface of the light homogenizing plate faces the eyes of the user.

In some embodiments, a distance between the light homogenizing plate and the broad-spectrum light source is 0 mm to 1 mm.

In some embodiments, the myopia illumination system further includes a power monitor, configured to control an emission power of the broad-spectrum light source within a preset range by monitoring the emission power.

In some embodiments, the power monitor implements the monitoring by receiving light that is emitted from the broad-spectrum light source and reflected by the smooth surface of the light homogenizing plate.

In some embodiments, the preset range is 0.1 mW to 1 W.

In some embodiments, the projection system includes a first aperture, an illuminating lens, a second aperture and a projecting lens arranged in sequence along an optical path, the second aperture being in close contact with the illuminating lens, in which the first aperture is optically conjugate with a light-entry position on an ocular surface of the eyes of the user; the second aperture is optically conjugate with a fundus spot of the eyes of the user; and a light-entry power of the myopia illumination system is determined based on one or more of: a beam waist size at the light-entry position, a size of the fundus spot, or an emission power of the broad-spectrum light source.

In some embodiments, in the myopia illumination system, in a case that a beam waist diameter does not exceed a specified threshold, the light-entry power is equal to the emission power of the broad-spectrum light source; or in a case that the beam waist diameter is greater than the specified threshold, the light-entry power is determined based on a power distribution of the emission power of the broad-spectrum light source and the size of the fundus spot.

In some embodiments, the specified threshold is 2.5 mm ± 0.5 mm.

In a second aspect, the disclosure provides a myopia illumination device. The myopia illumination device includes: the myopia illumination system according to any one embodiment of the first aspect.

In some embodiments, an inner diameter of an annular spot output by the myopia illumination system is adjusted based on measurement data of eyes of a user.

In some embodiments, the myopia illumination device further includes: a power controller, in which the power controller is communicatively connected to an external ocular measurement device, and is configured to obtain the measurement data of the eyes of the user, and control, based on the measurement data, an emission power of a broad-spectrum light source to be a specified emission power or one of a plurality of preset emission powers, and update the specified emission power or the preset emission power.

In some embodiments, the myopia illumination device further includes: a left eyepiece barrel and a right eyepiece barrel, in which the left eyepiece barrel and the right eyepiece barrel each are provided with the myopia illumination system, and the left eyepiece barrel and the right eyepiece barrel are connected by a spacing adjuster, and the spacing adjuster is configured to adapt to different pupil distances by adjusting a distance between the left eyepiece barrel and the right eyepiece barrel.

In some embodiments, the myopia illumination device further includes: a spacing controller, in which the spacing controller is connected to the spacing adjuster and is configured to control the spacing adjuster to adjust to a specified gear position or to cyclically move based on a preset mode.

In some embodiments, a 3D camera device is provided on the left eyepiece barrel and/or the right eyepiece barrel, configured to collect pupil information of the user.

In some embodiments, the myopia illumination device further includes: an optical axis controller and a mechanical adjuster; in which the optical axis controller is respectively connected to the mechanical adjuster and the 3D camera device, and is configured to control, based on the pupil information, the mechanical adjuster to adjust an axis of an optical path of the myopia illumination system.

In some embodiments, the myopia illumination device further includes: a shaping controller; in which the shaping controller is respectively connected to the 3D camera device and the beam shaping system, and is configured to control, based on the pupil information, the beam shaping system to form a beam waist diameter of the annular light entering the eyes that matches a pupil size.

In some embodiments, device data of the myopia illumination device is managed in a unified manner by a device management platform, and the myopia illumination device performs data interaction with the device management platform via wireless communication.

In some embodiments, a vibration motor is provided on a housing of the myopia illumination device, and the vibration motor is activated in a case that the myopia illumination system operates.

In a third aspect, the disclosure provides a control method for a myopia illumination device. The myopia illumination device includes: a broad-spectrum light source, a beam shaping system, a projection system and a shaping controller; a broad-spectrum light emitted by the broad-spectrum light source is shaped by the beam shaping system, and annular light formed by shaping is projected by the projection system onto eyes of a user to form an annular spot; in which the control method performed by the shaping controller includes: obtaining measurement data of the eyes of the user; determining a diameter of a macular fovea of the user based on the measurement data; and controlling the beam shaping system to shape the broad-spectrum light into specified annular light, in which an inner diameter of the specified annular light is consistent with the diameter of the macular fovea of the user.

In some embodiments, the myopia illumination device further includes: a power controller, and after obtaining the measurement data of the eyes of the user, the control method performed by the power controller includes: controlling, based on the measurement data, an emission power of the broad-spectrum light source to be a specified emission power or one of a plurality of preset emission powers; and/or, updating the specified emission power or the preset emission power based on the measurement data, in which the measurement data includes measurement data obtained in a plurality of periods.

In some embodiments, the myopia illumination device further includes: a left eyepiece barrel, a right eyepiece barrel, a spacing adjuster provided between the left eyepiece barrel and the right eyepiece barrel, and a spacing controller; the control method performed by the spacing controller includes: controlling, based on a pupil distance of the user, the spacing adjuster to adjust to a specified gear position or to cyclically move based on a preset mode.

In some embodiments, the myopia illumination device further includes: a mechanical adjuster for adjusting an axis of an optical path of the myopia illumination system, and an optical axis controller; in which the control method performed by the optical axis controller includes: controlling, based on pupil information of the user, the mechanical adjuster to adjust the axis of the optical path of the myopia illumination system and align with a pupil center of the user.

In some embodiments, the myopia illumination device further includes: a visual target system, configured to generate light of a visual target pattern, in which the light generated is projected onto the eyes of the user together with the annular light; in which the control method performed by the optical axis controller includes: controlling, based on the pupil information of the user, the mechanical adjuster to adjust a direction of the light of the visual target pattern and align with the pupil center of the user.

In some embodiments, the visual target system includes: a visual target light source and a beam splitter, an output optical path of the visual target light source coinciding with a first optical path of the beam splitter, and a second optical path of the beam splitter coinciding with a propagation optical path of the annular light; in which the control method performed by the optical axis controller further includes: controlling, based on the pupil information of the user, the mechanical adjuster to adjust one or more of: a position of the visual target light source, a position of the beam splitter, or an angle of the beam splitter.

In some embodiments, the myopia illumination device further includes: a left eyepiece barrel and a right eyepiece barrel, the left eyepiece barrel and the right eyepiece barrel each being provided with a myopia illumination system; and after obtaining the measurement data of the eyes of the user, the control method further includes: controlling, based on the measurement data, one or both of the myopia illumination systems in the left eyepiece barrel and the right eyepiece barrel to be turned on; and/or, controlling, based on the measurement data, a turn-on duration of the myopia illumination system in the left eyepiece barrel and a turn-on duration of the myopia illumination system in the right eyepiece barrel; and/or, controlling, based on the measurement data, an emission power of the myopia illumination system in the left eyepiece barrel and an emission power of the myopia illumination system in the right eyepiece barrel; in which the measurement data includes myopia diopters and/or axial length information of the eyes of the user.

The myopia illumination system adopts the broad-spectrum light source, which may improve the irradiation safety and avoid the risk of stimulating the fundus with the high power. In some embodiments, by performing beam shaping on the broad-spectrum light via the beam shaping system, the annular light may be irradiated to the eyes to form an annular spot, thus avoiding the position of the fovea of the eyes and improving the irradiation safety. In some embodiments, by designing the size of the annular spot, the system may adapt to a pupil size of the human eye and a macular center size, thus improving the illumination efficiency. Further, in some embodiments, the broad-spectrum light source is at least one of an LED lamp, an incandescent lamp, or a fluorescent lamp, which improves the safety and effectiveness for the human eye. In addition, in some embodiments, by performing spectrum shaping on the broad-spectrum light via the spectrum shaping system, light in the selected wavelength range may be irradiated to the fundus, thus achieving the desired effect.

### BRIEF DESCRIPTION OF THE DRAWINGS

The above and other objects, features and advantages of exemplary embodiments of the disclosure may become readily understood by reading the following detailed description with reference to the accompanying figures. In the accompanying figures, several embodiments of the disclosure are shown by way of examples rather than limitations, and the same or corresponding reference numerals denote the same or corresponding parts.
FIG. 1 is an exemplary structural diagram illustrating a myopia illumination system according to some embodiments of the disclosure.
FIG. 2 is a schematic diagram illustrating an exemplary implementation of a beam shaper according to some embodiments of the disclosure.
FIG. 3 is a schematic diagram illustrating a distribution of different tissue regions of the fundus.
FIG. 4 is a schematic diagram illustrating an annular spot according to some embodiments of the disclosure.
FIG. 5 is a schematic diagram illustrating an annular spot with a visual target pattern according to some embodiments of the disclosure.
FIG. 6 is an exemplary structural diagram illustrating a myopia illumination system according to some embodiments of the disclosure.
FIG. 7 is an exemplary structural diagram illustrating a myopia illumination system according to other embodiments of the disclosure.
FIG. 8a is a schematic diagram illustrating a spectrum shaper implemented by a filter.
FIG. 8b is a schematic diagram illustrating a spectrum shaper implemented by a transmission grating filter.
FIG. 8c is a schematic diagram illustrating a spectrum shaper implemented by a reflection grating filter.
FIG. 9 is a schematic diagram illustrating spectrum shaping of a spectrum shaper according to some embodiments of the disclosure.
FIG. 10 is an exemplary implementation diagram illustrating a spectrum shaping system including a plurality of spectrum shapers according to some embodiments of the disclosure.
FIG. 11 is a schematic diagram illustrating spectrum shaping of a spectrum shaper according to other embodiments of the disclosure.
FIG. 12 is an exemplary structural diagram illustrating a myopia illumination system according to other embodiments of the disclosure.
FIG. 13a is a schematic diagram illustrating a beam waist according to some embodiments of the disclosure.
FIG. 13b is a schematic diagram illustrating a beam waist according to other embodiments of the disclosure.
FIG. 14 is a schematic structural diagram illustrating a myopia illumination device according to some embodiments of the disclosure.
FIG. 15 is a schematic diagram illustrating a comparison between power distributions before and after operation of a vibration motor according to some embodiments of the disclosure.
FIG. 16 is an exemplary flowchart illustrating a control method for a myopia illumination device according to some embodiments of the disclosure.
FIG. 17 is an exemplary flowchart illustrating a control method for a myopia illumination device according to other embodiments of the disclosure.

### DETAILED DESCRIPTION

The technical solutions in embodiments of the disclosure will be clearly and completely described below with reference to the accompanying figures in the embodiments of the disclosure. It is apparent that the described embodiments are merely a part of, but not all of, embodiments of the disclosure. Based on the embodiments in the disclosure, all other embodiments obtained by those skilled in the art without inventive steps shall fall within the scope of protection of the disclosure.

It should be understood that the terms "comprises" and "includes" used in the specification and claims of the disclosure indicate the presence of described features, integers, steps, operations, elements, and/or components, but do not preclude the presence or addition of one or more other features, integers, steps, operations, elements, components, and/or groups of the described features, integers, steps, operations, elements, and/or components.

It should also be understood that the terms used in the specification of the disclosure are only for the purpose of describing specific embodiments and are not intended to limit the disclosure. As used in the specification and claims of the disclosure, the singular forms "a," "an," and "the" are intended to include the plural forms, unless the context clearly indicates otherwise. It should be further understood that the term "and/or" used in the specification and claims of the disclosure refers to any combination and all possible combinations of one or more of the associated listed items, and includes the combinations.

The specific embodiments of the disclosure are described in detail below with reference to the accompanying figures.

To avoid the risks that may be caused by a laser diode (LD), in the embodiments of the disclosure, a broad-spectrum light source is used to replace a narrow-spectrum LD, thus reducing the possible risk of stimulating the fundus with the high power. Specifically, the disclosure provides a myopia illumination system, including: a broad-spectrum light source, configured to emit broad-spectrum light; and a projection system, configured to project the broad-spectrum light onto eyes of a user. Based on the myopia illumination system, the disclosure provides various embodiments to further optimize the functions and effects of the system.

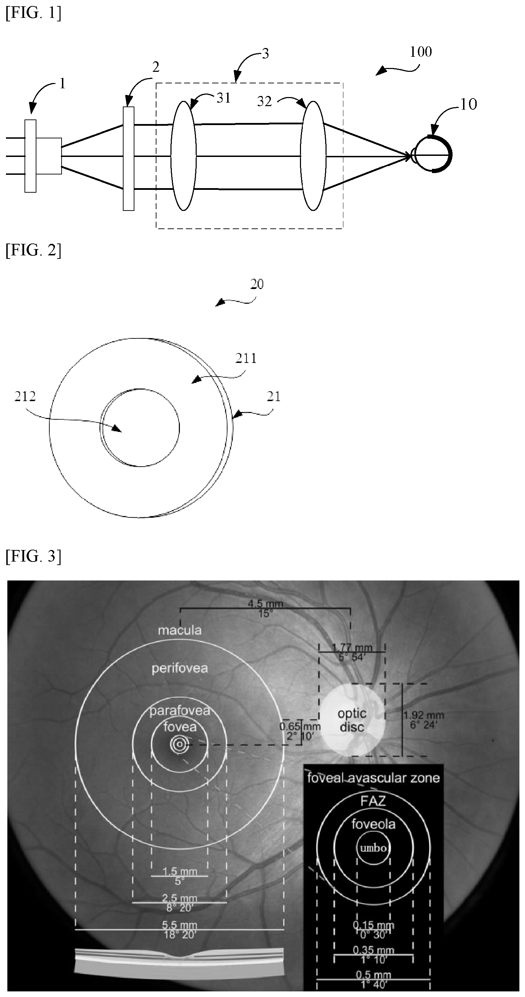

FIG. 1 is an exemplary structural diagram illustrating a myopia illumination system 100 according to some embodiments of the disclosure. As shown in FIG. 1, the myopia illumination system 100 may include: a broad-spectrum light source 1, a beam shaping system 2, and a projection system 3. The broad-spectrum light source 1 is configured to emit broad-spectrum light. The beam shaping system 2 is configured to shape the broad-spectrum light to form annular light. The projection system 3 is configured to project the annular light onto the eyes of the user to form an annular spot.

The broad-spectrum light source 1 may be arranged at an entrance end of a light transmission channel and is configured to emit the broad-spectrum light. In some embodiments, a spectral range of the broad-spectrum light source 1 may be a spectral range covering any spectral cutoff width within a range of 300 nm to 700 nm. In an example, the spectral range of the broad-spectrum light source is 500 nm to 800 nm, which covers a range of 500 nm to 700 nm. By using the broad-spectrum light source, rich spectral resources may be provided for the myopia illumination system, thus facilitating selection of light with the desired spectrum to irradiate the fundus retina, so as to achieve the desired effect. In some embodiments, the broad-spectrum light source 1 may include, but is not limited to, a light-emitting diode (LED) lamp, an incandescent lamp, a fluorescent lamp, etc. An emission power of the broad-spectrum light source 1 may be approximately 0.1 mW to 1 W, and an output power before entering the eyes may be approximately 0.1 mW to 15 mW. By using the light source and controlling the output power before entering the eyes, the safety of light irradiation may be improved during use of the myopia illumination system.

The beam shaping system 2 is configured to shape the broad-spectrum light emitted by the broad-spectrum light source 1 to form annular light. The broad-spectrum light source 1 may be generally regarded as a point light source, or a surface light source after homogenization. The shape of the broad-spectrum light emitted by the broad-spectrum light source 1 is generally circular. The circular light beam may be adjusted to the desired shape by the beam shaping system 2. For example, the circular light beam may be adjusted to annular light, thus being irradiated onto the eyes of the user in the desired shape. The specific implementation of the beam shaping system is described later.

The projection system 3 is configured to project the shaped annular light onto the eyes of the user, for example, the fundus of the eyes, and form an annular spot on the fundus. The projection system 3 may be implemented in various ways.

In some embodiments, the projection system 3 may include an illuminating lens 31 and a projecting lens 32. The illuminating lens 31 may be arranged on a light emitting side of the broad-spectrum light source 1 to collect the light emitted by the broad-spectrum light source 1 and project the emitted light to the projecting lens 32. The projecting lens 32 may be spaced apart from the illuminating lens 31 to project the light emitted by the illuminating lens 31 to a pupil 10 of the user.

In the disclosure, the lens refers to an optical element made of a transparent substance and having a surface that is part of a spherical surface. The terms "illuminating" and "projecting" are functional descriptions, and specific implementations of the illuminating lens and the projecting lens may be a single lens or a lens group composed of a plurality of lenses. The illuminating lens and the projecting lens may form a system structure of Köhler illumination. Those skilled in the art may understand that the projection system may also be implemented in other ways, such as critical illumination projection, optical lens projection, optical waveguide projection, AR/VR projection systems, or may be illumination systems such as direct light source illumination, LED desk lamp illumination, indoor or outdoor lamp illumination. However, the inventive concept of the disclosure does not reside therein, and thus a detailed description of the projection system is omitted.

Thus, with the myopia illumination system provided above, the broad-spectrum light source is adopted, and the beam shaping system performs beam shaping on the broad-spectrum light, so that the annular light may be irradiated onto the eyes of the user to form the annular spot, thus avoiding the position of the macular fovea of the eyes and improving the irradiation safety. In some embodiments, the broad-spectrum light source adopts an LED, which may improve the safety and effectiveness for the human eye.

As mentioned above, the beam shaping system may perform beam shaping on the broad-spectrum light, thus forming a light beam of a specific shape, for example, annular light. The beam shaping system may include one or more beam shapers, and thus light of various shapes and specifications may be formed. The beam shaper may be implemented in various ways.

FIG. 2 is a schematic diagram illustrating an exemplary implementation of a beam shaper according to some embodiments of the disclosure. In the embodiments, the beam shaper may shape incident circular light into annular light.

As shown in FIG. 2, the beam shaper 20 includes a substrate 21. The substrate 21 includes an annular transparent portion 211 and an opaque portion 212 located at a center of the annular transparent portion. When the circular light is incident on the substrate 21, the light is transmitted to only pass through the annular transparent portion 211, thus forming the annular light.

In other embodiments, the beam shaper 20 may also have a spectrum shaping function, and the beam shaper 20 may also be understood as a spectrum shaper, for example, a filter, etc., and spectrum shaping is described in detail later. Referring to the beam shaper shown in FIG. 2, the spectrum shaper may also include an annular transparent portion and an opaque portion located at a center of the annular transparent portion. In some embodiments, the opaque portion may be circular or of other shapes, for example, a hexagon or an octagon. As an example, the shape of the opaque portion in the beam shaper 20 is preferably circular to match the shape of the macular fovea.

The material of the annular transparent portion may include any one of: glass, crystal, plastic, or resin. Further, the plastic/resin may include any one of: polymethyl methacrylate (PMMA), polystyrene (PS), cyclic olefin copolymer (COC), cyclic olefin polymer (COP), rigid polyethylene terephthalate (PET), polyvinyl chloride (PVC), or polycarbonate (PC). It should be noted that the above materials are only some examples provided in the disclosure, and other plastic or resin materials may also be used in practical applications, which are not listed one by one here. The material of the opaque portion is an opaque material, or the opaque portion is made of the same material as the annular transparent portion but is subjected to an opaque treatment, for example, sandblasting or painting, and the treated surface is opaque.

In an example, the beam shaper 20 may use an acrylic transparent plate as the substrate, a circular groove is excavated at the center, and then black acrylic matching the groove is embedded, in which the black acrylic is opaque. Similarly, the spectrum shaper may be, after excavating a circular groove at the center, embedded with the black acrylic matching the groove, in which the black acrylic is opaque.

In another example, the beam shaper 20 may use an acrylic transparent plate as the substrate, sandblasting or painting is performed within a circular area at the center, or a black-dot annular transparent film is attached at the center, and the treated circular surface is opaque. Similarly, the spectrum shaper may be subjected to sandblasting, painting, or attaching the black-dot annular transparent film within the circular area at the center, thus completing the opaque treatment.

In another example, the beam shaper 20 uses rigid PET, PVC, or PC as the substrate, sandblasting or painting is performed within the circular area at the center, or the black-dot annular transparent film is attached at the center, and the treated surface is opaque.

The annular light formed after the beam shaping may be projected onto the eyes of the user via the projection system to form an annular spot, thus avoiding the position of the fovea of the eyes and improving the irradiation safety.

FIG. 3 is a schematic diagram illustrating distribution of different tissue regions of fundus. It may be seen from FIG. 3 that a diameter of fovea is about 1.5 mm, a diameter of parafovea is about 2.5 mm, and a diameter of perifovea is about 5.5 mm. The figure further shows the size of the foveal avascular zone (FAZ), a diameter of the FAZ is about 0.5 mm, a diameter of foveola is about 0.35 mm, and a diameter of umbo is about 0.15 mm. Based on the distribution of different tissue regions on the fundus, the size and shape of the annular spot may be designed to irradiate the desired region while avoiding irradiation of the undesired region.

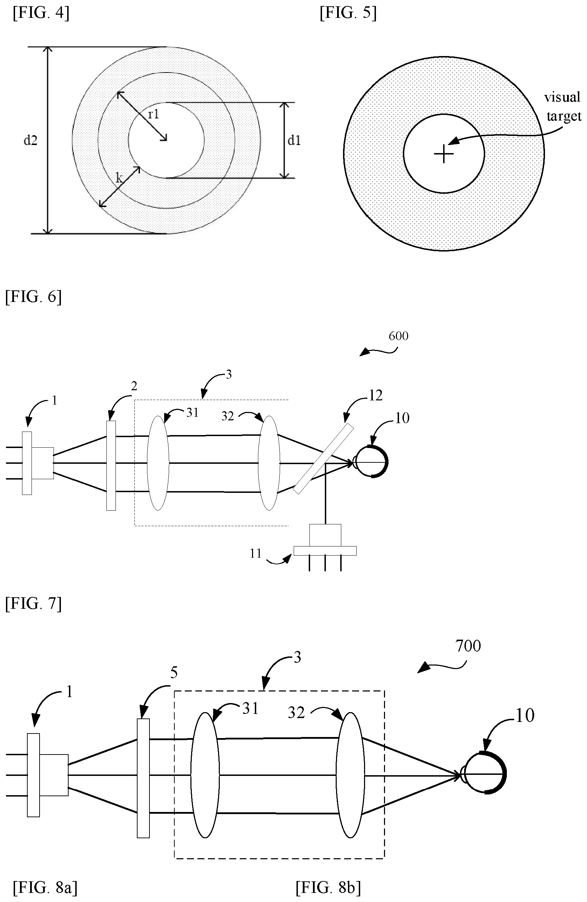

FIG. 4 is a schematic diagram illustrating an annular spot according to some embodiments of the disclosure. The size of the annular spot may be represented by a plurality of parameters. As shown in FIG. 4, in an example, the size of the annular spot may be represented by an inner diameter d1 of an inner ring and an outer diameter d2 of an outer ring. In consideration of the distribution of different tissue regions of the fundus, the inner diameter d1 of the annular spot output by the myopia illumination system is between 0.0076 mm and 6 mm. In some embodiments, the size of the annular spot may be any one of following combinations that:
the inner diameter d1 is about 1.5 mm, and the outer diameter d2 is about 2.5 mm, and in this case, the annular spot irradiates the parafovea region but avoids the fovea region;
the inner diameter d1 is about 2.5 mm, and the outer diameter d2 is about 5.5 mm, and in this case, the annular spot irradiates the perifovea region but avoids the parafovea region; or
the inner diameter d1 is about 1.5 mm, and the outer diameter d2 is about 5.5 mm, and in this case, the annular spot irradiates the perifovea region and only avoids the fovea region.

In another example, the size of the annular spot may be represented by an annular width k and a distance r1 from a spot center to an annular centerline. In some embodiments, the annular width k of the annular spot may be about 0.0038 mm to 3 mm, and the distance r1 from the spot center to the annular centerline may be about 0.0038 mm to 3 mm.

By designing the size of the annular spot, the system may adapt to a pupil size of the human eye and a macular center size, thus improving the illumination efficiency. The size of the annular spot may be achieved by selecting an appropriate beam shaper, adjusting relevant optical devices (such as apertures, etc.) in the myopia illumination system. Regardless of which means are used for adjustment, it is only necessary to ensure that the annular spot finally projected onto the eyes of the user meets the preset size requirement.

Since the annular spot output by the myopia illumination system is designed based on the tissue structure of the eyes of the user, the annular spot needs to be aligned with the eye position of the user during use, so that the annular spot is projected at a matching position, ensuring the illumination effect. To assist the alignment, optionally or additionally, in some embodiments, a visual target pattern may be added into the annular spot output by the myopia illumination system. FIG. 5 is a schematic diagram illustrating an annular spot with a visual target pattern according to some embodiments of the disclosure. The visual target pattern is a fixation point, guiding the pupil of the user to stably align with the visual target pattern, thus ensuring the angle and the position at which the annular spot enters the pupil.

In some embodiments, in order to form the annular spot with the visual target pattern, a visual target system is additionally provided in the myopia illumination system. The visual target system is configured to generate light of the visual target pattern, in which the light generated is projected onto the eyes of the user together with the annular light. In an example, the visual target pattern may be formed by the beam shaping system. A visual target transparent portion is provided on the beam shaper or the spectrum shaper shown in FIG. 2, the visual target transparent portion is arranged at the center of the opaque portion, and is configured to form the annular light with the visual target pattern at the center by transmitting a portion of the broad-spectrum light. It should be noted that the shape of the visual target transparent portion is not strictly limited in the embodiment, and the shape of the visual target transparent portion may be a circle, a ring, a cross, or a star, which is not limited herein.

In another example, the visual target pattern may be formed by an additional visual target light source and a beam splitter. FIG. 6 is an exemplary structural diagram illustrating a myopia illumination system 600 according to some embodiments of the disclosure. As shown in FIG. 6, the myopia illumination system may further include a visual target light source 11 and a beam splitter 12. The broad-spectrum light source 1, the beam shaping system 2 and the projection system 3 are arranged on the same optical path, and the visual target light source 11 is arranged on another optical path. The beam splitter 12 is arranged at an intersection of the two optical paths, in which the optical path where the broad-spectrum light source 1 is located is a first optical path, and the optical path where the visual target light source 11 is located is a second optical path. The beam splitter 12 may transmit the light of the first optical path and reflect the light of the second optical path, thus projecting the annular spot and the visual target spot onto the fundus of the user together. The position where the visual target spot is projected onto the fundus is specifically located at the center of the annular spot.

It should be noted that the beam splitter 12 may also reflect the light of the first optical path and transmit the light of the second optical path, thus projecting the annular spot and the visual target spot onto the fundus of the user together. It may be understood that the optical path where the broad-spectrum light source 1, the beam shaping system 2 and the projection system 3 are located in FIG. 6 may be swapped with the optical path where the visual target light source 11 is located, which is not limited herein.

It should be further noted that the shape of the visual target spot is not strictly limited in the embodiment. In some embodiments, the shape of the visual target light may be adjusted by some ways such as adjusting the shape of a light exit port of the visual target light source, for example, by providing a light shielding plate with a specific shape at the light exit port of the visual target light source, thus achieving visual target spots of different shapes.

FIG. 7 is an exemplary structural diagram illustrating a myopia illumination system 700 according to other embodiments of the disclosure. As shown, the myopia illumination system 700 may include: a broad-spectrum light source 1, a spectrum shaping system 5, and a projection system 3. The broad-spectrum light source 1 is configured to emit broad-spectrum light. The spectrum shaping system 5 is configured to form light with a specified spectrum by shaping a spectrum of the broad-spectrum light. The projection system 3 is configured to project the light with the specified spectrum onto the eyes of the user. For the broad-spectrum light source and the projection system, reference may be made to the above description in conjunction with FIG. 1, which is not repeated herein.

In the embodiments of the disclosure, spectrum shaping refers to adjusting an optical spectrum of incident light, for example, adjusting a wavelength range of the light, specifically including a start wavelength, a cutoff wavelength, a spectral cutoff width, and the like. By performing spectrum shaping on the broad-spectrum light, light of the desired wavelength may be selected from the broad-spectrum light to irradiate the fundus, thus achieving the desired effect.

The spectrum shaping system may include one or more spectrum shapers. The spectrum shaper may be implemented in various ways.

FIG. 8a to FIG. 8c are schematic diagrams illustrating exemplary implementations of a spectrum shaper according to some embodiments of the disclosure.

FIG. 8a is a schematic diagram illustrating a spectrum shaper implemented by a filter. In the embodiment, the spectrum shaper may be implemented by a single filter or a plurality of filters. The figure shows the principle of spectrum shaping by the filter. As shown in FIG. 8a, the filter 51 selectively transmits a part of the spectrum of incident light 511, and blocks transmission of a remaining part of the spectrum via absorption, reflection, etc. That is, specified light 512 is transmitted, and unspecified light 513 is reflected and absorbed based on spectral characteristics of the filter.

FIG. 8b is a schematic diagram illustrating a spectrum shaper implemented by a transmission grating filter. In the embodiment, the spectrum shaper may be implemented by a single transmission grating filter or a plurality of transmission grating filters. The figure shows an exemplary structure of the transmission grating filter. As shown in FIG. 8b, the transmission grating filter 52 is composed of a series of parallel equally spaced slits or protrusions. When the light of the light source passes through the grating in parallel, a diffraction effect is produced. By changing grating parameters, light of the specific wavelength may pass through the grating, while light of remaining wavelengths may be diffracted to other places or eliminated, thus achieving the effect of spectrum shaping.

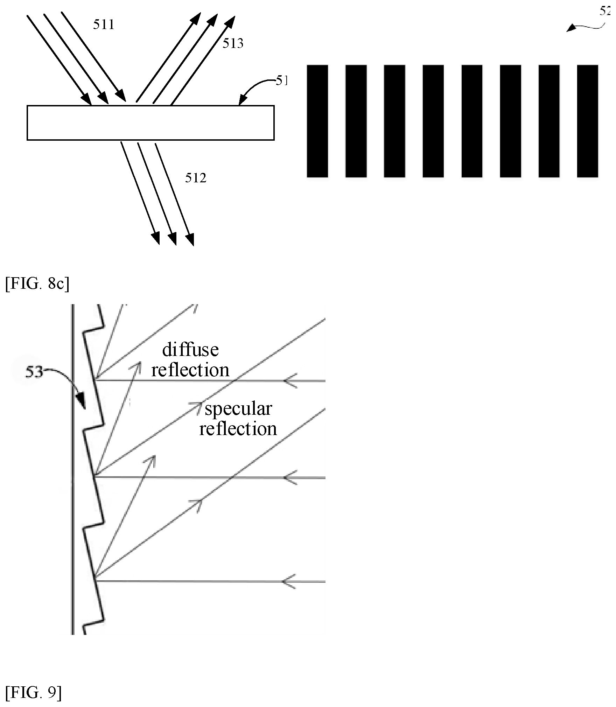

FIG. 8c is a schematic diagram illustrating a spectrum shaper implemented by a reflection grating filter. In the embodiment, the spectrum shaper may be implemented by a single reflection grating filter or a plurality of reflection grating filters. The figure shows the principle of spectrum shaping by the reflection grating filter. As shown in FIG. 8c, on the reflection grating filter 53, light of the specific wavelength undergoes specular reflection and is collected by the system, and light of unselected wavelengths undergoes diffuse reflection or is eliminated, thus achieving the effect of spectrum shaping.

Those skilled in the art may understand that other devices may also be used to implement spectrum shaping. For example, the spectrum shaper may be formed by a single optical element or a plurality of different optical elements, and the optical elements include, but are not limited to, a prism, a lens, etc. For another example, the spectrum shaper may be formed by a non-optical element, such as a liquid crystal spectrum transmission plate; or a spectrum absorber including a spectrum absorbing material, etc. Regardless of the form taken, the essence of the spectrum shaper is an optical filter. Thus, a spectral range of the spectrum shaper implemented by various devices may be represented by filtering parameters of the optical filter. The filtering parameters may include, but are not limited to: a central wavelength, a start wavelength (also referred to as a low-pass cutoff wavelength), a cutoff wavelength (also referred to as a high-pass cutoff wavelength), a spectral cutoff width, etc. The spectral characteristics of the spectrum shaper may similarly include any one of: high-pass; low-pass; band-pass; or multi-band-pass. Each spectrum shaper may have its own spectral range and spectral characteristics.

FIG. 9 is a schematic diagram illustrating spectrum shaping of a spectrum shaper according to some embodiments of the disclosure. In the embodiment, a spectral range of the spectrum shaper may be that, a low-pass cutoff wavelength range is approximately 640 nm to 660 nm, and a high-pass cutoff wavelength range is approximately 670 nm to 700 nm, that is, a spectral cutoff width is approximately 10 nm to 60 nm. As shown in FIG. 9, a transmission spectrum range is a spectrum range irradiating the retina of the fundus. The spectral range of the embodiment may correspond to a red light spectrum.

Through research, it has been found that using a wavelength of 650 nm has a good effect on myopia prevention and control for adolescents. In addition, it has also been found that using a wavelength of 670 nm has a related effect on myopia prevention and control for middle-aged people. The prevention and control principle lies in that, the wavelength band where the long-wave red light is located has strong penetration, so red light may act on the choroid after penetrating the retina. Since the red light has a warm-heat effect, the bottleneck-like stenosis at the opening of the small artery of the choroidal lobule may be opened, which increases the blood flow entering the lobule, increases the microcirculation blood volume, thickens the choroid, and prevents scleral hypoxia. Providing sufficient oxygen for the sclera may improve blood circulation of the fundus, thus achieving the effect of myopia prevention and control. In addition, the red light of 650 nm may enable retinal pigment epithelial cells to secrete dopamine, thus effectively inhibiting excessive growth of the axial length. Thus, by adjusting parameters such as the low-pass cutoff wavelength and the high-pass cutoff wavelength of the spectrum shaper based on the above range, the suitable red light may be selected from the broad-spectrum light source to match the conditions of the user, so as to obtain good effects of myopia prevention and control.

Further, in some embodiments, the spectrum shaping system may include a plurality of spectrum shapers, and each spectrum shaper may have a different spectral range and/or a different spectral cutoff width, thus achieving free combination of different spectral ranges and different spectral cutoff widths. The combination mode may effectively improve a utilization rate of the spectrum and an effect of myopia prevention and control and improvement.

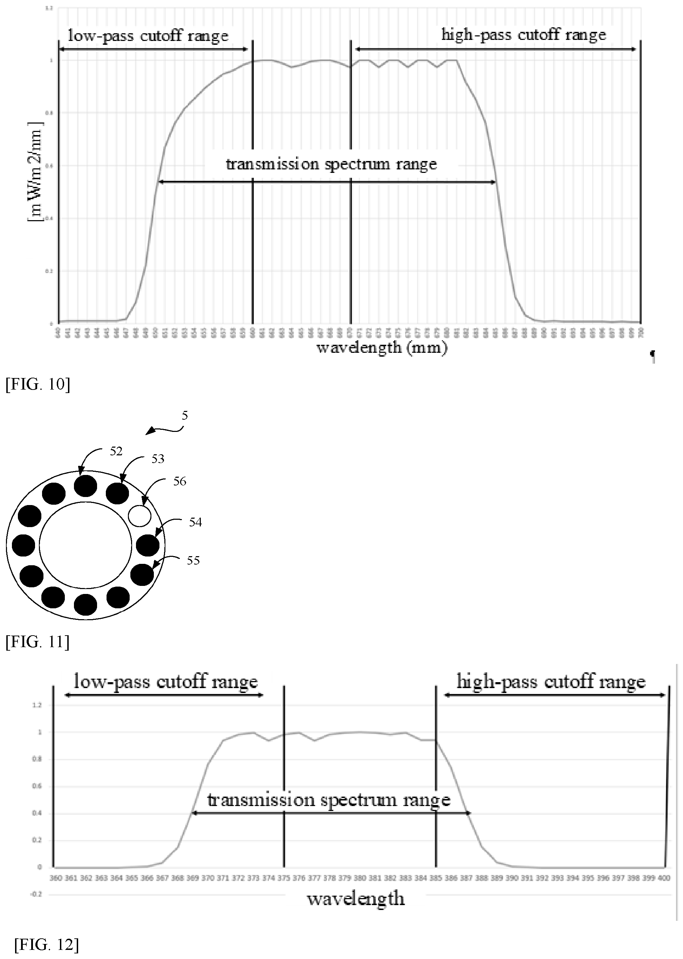

FIG. 10 is an exemplary implementation diagram illustrating a spectrum shaping system including a plurality of spectrum shapers according to some embodiments of the disclosure. As shown in FIG. 10, the spectrum shaping system 5 may be implemented as a rotatable wheel, and a plurality of spectrum shapers (for example, filters) distributed circumferentially are arranged at an edge of the wheel, in which the plurality of spectrum shapers include at least two different spectrum shapers. For example, the figure shows a spectrum shaper 52 with a spectral cutoff width of 10 nm and a spectrum shaper 53 with a spectral cutoff width of 20 nm, which have the same central wavelength. The figure further shows a spectrum shaper 54 with a central wavelength of 650 nm and a spectrum shaper 55 with a central wavelength of 670 nm, which have the same spectral cutoff width, thus being capable of adapting to different types of users. In addition, the figure further shows a transparent plate 56, which may be regarded as an all-pass band spectrum shaper. It may be understood that other spectrum shapers in the figure may have different central wavelengths and spectral cutoff widths, which are not listed one by one here.

FIG. 11 is a schematic diagram illustrating spectrum shaping of a spectrum shaper according to other embodiments of the disclosure. In the embodiment, a spectral range of the spectrum shaper may be that, a low-pass cutoff wavelength range is approximately 360 nm to 375 nm, and a high-pass cutoff wavelength range is approximately 385 nm to 400 nm, that is, a spectral cutoff width is approximately 10 nm to 40 nm. As shown in FIG. 11, a transmission spectrum range is a spectrum range irradiating the retina of the fundus. The spectral range of the embodiment may correspond to a violet light spectrum.

Through research, it has been found that the violet light may also have many effects on eye health and myopia prevention and control. Thus, by adjusting parameters such as the low-pass cutoff wavelength and the high-pass cutoff wavelength of the spectrum shaper based on the above range, the suitable violet light may be selected from the broad-spectrum light source to irradiate the fundus of the user, so as to obtain the desired effect of myopia prevention and control.

Similarly, in some embodiments, the spectrum shaping system may include a plurality of spectrum shapers, and each spectrum shaper may have a different spectral range and/or a different spectral cutoff width, thus achieving free combination of different spectral ranges and different spectral cutoff widths.

The red light spectrum shaping and the violet light spectrum shaping may be arranged separately or in combination, for example, a combination of different spectral cutoff widths in the red light spectrum, a combination of different spectral cutoff widths in the violet light spectrum, or a combination of the red light spectrum and the violet light spectrum. The spectral ranges of different spectrum shapers may be the same or different, may partially overlap, or may have no overlap. Thus, the utilization rate of the spectrum and the effect of myopia prevention and control and improvement may be effectively improved.

In some embodiments, a spectral cutoff width of a single spectrum shaper is about 5 nm to 60 nm, and a central wavelength may be selected from any wavelength within a wavelength range of 360 nm to 400 nm or within a wavelength range of 640 nm to 700 nm. The spectral cutoff width may be, for example, 60 nm, 40 nm, 20 nm, 10 nm, 5 nm, etc. Narrowing the spectral cutoff width may accurately select the desired wavelength, ensure purity of the narrow-band spectrum, and obtain an improvement effect brought by the corresponding wavelength.

In other embodiments, a combination of a plurality of spectrum shapers may be adopted, thus providing a plurality of spectral ranges for selection. For example, a spectral range of a first spectrum shaper is: 380±10 nm, a spectral range of a second spectrum shaper is: 650 nm±5 nm, a spectral range of a third spectrum shaper is: 665 nm±5 nm, a spectral range of a fourth spectrum shaper is: 670 nm±5 nm, a spectral range of a fifth spectrum shaper is: 680 nm±5 nm, a spectral range of a sixth spectrum shaper is: 650 nm±10 nm, a spectral range of a seventh spectrum shaper is: 665 nm±10 nm, a spectral range of an eighth spectrum shaper is: 665 nm±20 nm, a spectral range of a ninth spectrum shaper is: 670 nm±20 nm, a spectral range of a tenth spectrum shaper is: 680 nm±20 nm, etc.

When a plurality of spectrum shapers are used in combination, the spectrum shapers may be of the same type. For example, the spectrum shapers are all filters, or are all reflection grating filters, etc. Alternatively, the spectrum shapers may be of different types. For example, some of the spectrum shapers are filters, some of the spectrum shapers are grating filters, some of the spectrum shapers are prisms, etc. Those skilled in the art may, under the descriptions of the disclosure, select an appropriate spectrum shaper based on a spatial structure of the myopia illumination system.

FIG. 12 is an exemplary structural diagram illustrating a myopia illumination system 1200 according to other embodiments of the disclosure. In the embodiments, some other components in the myopia illumination system are further shown to better understand the solution of the disclosure. Based on the myopia illumination system 100 shown in FIG. 1, the myopia illumination system 1200 of FIG. 12 further includes a light homogenizing member, a condenser lens, an aperture, a color temperature adjuster, a power monitor, and/or a spectrum shaping system, etc.

As shown in FIG. 12, the myopia illumination system 1200 may include: a broad-spectrum light source 1, a power monitor 4, a light homogenizing element 6, a condenser lens 7, a beam shaping system 2, an aperture 8, a color temperature adjuster 9, a spectrum shaping system 5, an illuminating lens 31, and a projecting lens 32, which are arranged along an optical path. The broad-spectrum light source 1 may be an LED, configured to emit a divergent light beam. After passing through a light homogenizing effect of the light homogenizing element 6 and a converging effect of the condenser lens 7, a converged uniform light beam is formed. The uniform light beam is shaped by the beam shaping system 2 to form annular light. Then, a color temperature adjustment may be performed by the color temperature adjuster 9, and spectrum shaping may be performed by the spectrum shaping system 5, so that an output light beam has a specified spectral range. During transmission of the light beam, the aperture 8 may adjust a size and/or power of the light beam passing through the aperture 8. The adjusted light beam passes through the illuminating lens 31 and the projecting lens 32, and is finally output as a uniformly distributed illumination light beam, which is projected onto the pupil 10 of the user.

Those skilled in the art should understand that FIG. 12 only shows an exemplary structure of the myopia illumination system. In the structure, the power monitor 4, the light homogenizing element 6, the condenser lens 7, the beam shaping system 2, the spectrum shaping system 5, the aperture 8, and the color temperature adjuster 9 are all optional optical elements. When configuring the myopia illumination system, one or more of the above elements may be selected and arranged in the myopia illumination system based on actual needs, or other optical elements may be added, such as the visual target light source 11 and the beam splitter 12 in the embodiment described above in conjunction with FIG. 6, to achieve corresponding functions.

In some embodiments, the light homogenizing element 6 is an element having the function of homogenizing/uniformizing the light from the light source, such as a light homogenizing plate, a diffusion sheet, a light guide plate, etc. Taking the light homogenizing plate as an example, the light homogenizing plate may be arranged at a position about 0 mm to 1 mm away from the broad-spectrum light source 1. The closer the distance between the light homogenizing plate and the broad-spectrum light source 1, the more energy of the light output from the broad-spectrum light source 1 passes through the light homogenizing plate, and the higher the utilization rate of the light source. As an example, the light homogenizing plate may be closely attached to a light exit port of the broad-spectrum light source 1, so that the light output from the broad-spectrum light source 1 all passes through the light homogenizing plate. In practical application, when inserting the light homogenizing plate into the myopia illumination system 1100, it is necessary to pay attention to selecting an appropriate distance spaced between the light homogenizing plate and the broad-spectrum light source 1, so as to ensure that an assembly tolerance does not cause the light homogenizing plate to be deformed or have other problems.

In some embodiments, a smooth surface of the light homogenizing plate faces the broad-spectrum light source 1. Since the light source only has penetration through the smooth surface, the smooth surface of the light homogenizing plate faces the broad-spectrum light source 1, which may ensure to the greatest extent that the energy passes through the light homogenizing plate. The rough surface of the light homogenizing plate faces the eyes of the user. Such arrangement may cause the light passing through the light homogenizing plate to undergo a scattering/diffusing effect on the rough surface of the light homogenizing plate, so as to form a uniform surface light source on the rough surface. It should be further noted that if the light homogenizing element adopts other optical elements, correspondingly, a surface of the optical element that has the scattering effect needs to face the eyes of the user, and the other surface of the optical element faces the broad-spectrum light source 1.

In addition, since the smooth surface of the light homogenizing plate faces the broad-spectrum light source 1 and may reflect the light at large angles, a power monitor 4 is arranged on a reflection path of the light to monitor an emission power of the broad-spectrum light source, thus facilitating controlling the emission power within a preset range. The preset range is, for example, approximately 0.1 mW to 1 W. As an example, the power monitor 4 may be a photoelectric conversion chip. The photoelectric conversion chip receives the light that is emitted from the broad-spectrum light source 1 and reflected by the smooth surface of the light homogenizing plate, collects energy of the light and converts the energy into an electrical signal. The electrical signal may be transmitted to the background for monitoring, thus achieving an effect of monitoring stability of the emission power of the broad-spectrum light source 1 and improving safety of the myopia illumination system.

As an example, when performing stability monitoring, taking the photoelectric conversion chip as an example, the photoelectric conversion chip outputs a Photodiode (PD) value, for example 100, and the PD value corresponds to a power of 0.1 mW. The background may determine the stability of the emission power of the broad-spectrum light source 1 by identifying a change in the PD value output by the photoelectric converter. In an embodiment, the background may monitor an absolute value of the PD value, and when the PD value exceeds an acceptable range, determine that the power is abnormal, issue an alarm and/or actively intervene to adjust the emission power of the broad-spectrum light source 1. In another embodiment, the background may monitor a fluctuation of the PD value over a period of time to determine the abnormality. For example, when an extreme value of the fluctuation of the PD value within a period of time reaches a fluctuation threshold, the background may determine that the power is abnormal, issue an alarm and/or actively intervene to adjust the emission power of the broad-spectrum light source 1. It should be noted that the above descriptions are examples of implementation of the monitoring function provided by the embodiment, and in practical applications, other implementations are also suitable for the disclosure.

In some embodiments, the broad-spectrum light source 1 is located at an entrance pupil plane of the condenser lens 7. Further, in some embodiments, the condenser lens 7 may be closely attached to a light exit surface of the broad-spectrum light source 1. Such arrangement may make a target surface of the broad-spectrum light source 1 be located at the entrance pupil plane of the condenser lens 7, thus making all the divergent light beam output by the broad-spectrum light source 1 converge.

The condenser lens 7 may be a plano-convex lens, an aspherical lens, or another optical element having a light converging function. Assuming a plano-convex lens is used as the condenser lens, a convex surface of the plano-convex lens faces the light source, and a plane surface of the plano-convex lens faces the eyes of the user. It should be further noted that if the condenser lens adopts another optical element, correspondingly, a surface of the optical element that diverges the light beam needs to face the light source, and another surface of the optical element that converges the light beam needs to face the eyes of the user, thus collecting the divergent light beam and performing converging processing.

Further, in the myopia illumination system provided with the power monitor 4, in a case that the power monitor 4 monitors a power abnormality, the emission power of the light source may also be adjusted by moving the condenser lens in a direction away from the light source.

In some embodiments, the spectrum shaping system 5 is configured to form light with a specified spectrum by shaping a spectrum of the broad-spectrum light from the broad-spectrum light source before the light is projected onto the eyes of the user. In the disclosure, spectrum shaping refers to adjusting an optical spectrum of incident light, for example, adjusting a wavelength range of the light, specifically including a start wavelength, a cutoff wavelength, a spectral cutoff width, and the like. By performing spectrum shaping on the broad-spectrum light, light of the desired wavelength may be selected from the broad-spectrum light to irradiate the fundus, thus achieving the desired effect.

The spectrum shaping system 5 may include one or more spectrum shapers. In the embodiment, the spectrum shaper may be implemented in various ways. As an example, the spectrum shaper may be implemented by a single filter or a plurality of filters. The implementations and shaping principles of the spectrum shaping system 5 have been described in detail in the embodiments described above in conjunction with FIG. 7a to FIG. 7c. For specific configurations and implementations, reference is made to the above description, which is not repeated herein.

Further, the plurality of spectrum shapers in the spectrum shaping system 5 may be switched via a switcher. By manipulating the switcher to insert a specified spectrum shaper into an optical path of the myopia illumination system, targeted spectrum shaping may be performed on the broad-spectrum light, and light of the desired wavelength may be selected from the broad-spectrum light to irradiate the fundus. It should be noted that any device capable of implementing the above switching function is applicable to the embodiment, for example, a rotary switcher (e.g., filter wheel), which is not limited in embodiments of the disclosure.

In some embodiments, the aperture 8 may be an aperture stop. By adjusting an aperture size parameter of the aperture stop, a size and/or power of the light beam may be adjusted. As an example, the aperture stop may be located at an exit pupil plane of the condenser lens, so that light from the divergent light source converged by the condenser lens may pass through the aperture stop, thus improving the power of light from the light source passing through the aperture stop. When the light source is an LED, since the LED light source has a large divergence angle and the power distribution is divergent, the divergent light beam output by the LED light source is converged after passing through the condenser lens, so that the power distribution of the light source is concentrated, which allows the light to propagate backward through the aperture stop. In this case, the light source at the aperture stop is equivalent to a surface light source, and the power attenuation is small. The divergence angle is a divergence angle of the converged light beam after passing through the aperture stop, and the size is an aperture size of the aperture stop.

In addition, the aperture stop is located at a front focal plane of the illuminating lens 31, and an exit pupil plane of the illuminating lens 31 is located between the illuminating lens 31 and the projecting lens 32 and close to the illuminating lens 31. That is, the exit pupil plane of the illuminating lens 31 is located behind the illuminating lens 31 and at a position close to a rear surface of the illuminating lens 31. Such arrangement may make energy distribution of a spot on the exit pupil plane of the illuminating lens 31 more uniform.

The exit pupil plane of the illuminating lens 31 is also located at a front focal plane of the projecting lens 32. With such arrangement, when the myopia illumination system is used, the spot distributed on the exit pupil plane of the illuminating lens 31 may be projected onto the fundus, so that the human eye sees a uniform surface light source. Further, it may be designed such that the pupil of the eyes of the user is located at a rear focal plane of the projecting lens 32, so that all the light emitted from each position on the exit pupil plane of the illuminating lens 31 may pass through the pupil and irradiate the fundus of the user.

Based on the above position conditions of the illuminating lens 31 and the projecting lens 32, those skilled in the art may clearly understand that, in the embodiment, the illuminating lens 31 and the projecting lens 32 form a system structure of Köhler illumination, thus solving the problem of non-uniformity of the light source. Those skilled in the art should understand that, in the embodiment, the projection system may also be implemented in other ways, for example, critical illumination projection, optical lens projection, optical waveguide projection, AR/VR projection systems, or may be illumination systems such as direct light source illumination, LED desk lamp illumination, indoor or outdoor lamp illumination, etc.

In other embodiments, the aperture 8 may include a first aperture and a second aperture, in which the first aperture, the illuminating lens 31, the second aperture and the projecting lens 32 are arranged in sequence and form a projection system. For the positions of the illuminating lens 31 and the projecting lens 32 in the projection system, reference may be made to the above description. For the position of the first aperture, reference may be made to the position of the aperture stop described above, which is not repeated herein.

As an example, the second aperture is in close contact with the illuminating lens 31, the first aperture is optically conjugate with a light-entry position on an ocular surface of the eyes of the user, and the second aperture is optically conjugate with a fundus spot of the eyes of the user. In this case, a beam waist size at an optically conjugate position of the first aperture and the light-entry position on the ocular surface of the eyes of the user depends on an aperture size of the first aperture, and the size of the fundus spot of the eyes of the user or an incident angle to the eye depends on an aperture size of the second aperture. It may be understood that, by adjusting the aperture size of the first aperture and/or the second aperture, the fundus spot projected onto the eyes of the user may be shaped, specifically, the size of the fundus spot may be shaped.

When the myopia illumination system is used to irradiate the eyes of the user, since the human eye is stimulated by light, the pupil automatically contracts, causing a pupil diameter to be reduced by about 2.5 mm, which leads to a difference between a power of the fundus spot output by the myopia illumination system and an actual light-entry power. Specifically, the actual light-entry power decreases by a square factor due to the pupil contraction, and a relational expression is exemplarily expressed as follows: w=W×(L-d)2/L2, in which w represents the actual light-entry power, W represents a spot power output by the myopia illumination system, L represents the pupil diameter, and d represents a pupil contraction amount. The pupil contraction of the eyes of the user narrows an entrance aperture of the eyes of the user, causing that an area of an entrance region is reduced by a square factor, thus losing the power by a square factor.

Further, a light-entry power of the myopia illumination system is determined based on one or more of: a beam waist size at the light-entry position, a size of a fundus spot, or an emission power of the broad-spectrum light source. It may be understood that, by adjusting an aperture size of the first aperture and/or the second aperture, a power distribution of the fundus spot projected onto the eyes of the user may also be shaped.

In order to ensure referability of the light-entry power and effectiveness of illumination, in some embodiments, the first aperture is designed to be optically conjugate with the light-entry position on the ocular surface of the eyes of the user, so that a light beam at the light-entry position on the ocular surface converges, and an incident angle of actual light entering the eyes is 1.5 mrad to 350 mrad.

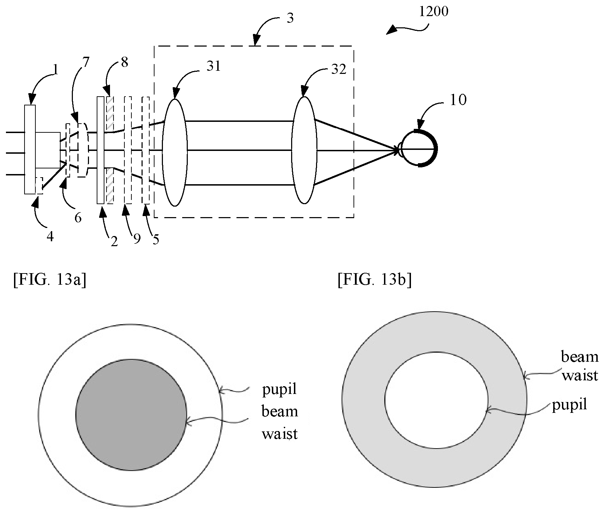

In some embodiments, the case that power adjustment is completed by controlling the beam waist size at the light-entry position on the ocular surface is as follows. In a case that a beam waist diameter does not exceed a specified threshold, the light-entry power is equal to the emission power of the broad-spectrum light source. In some embodiments, the specified threshold is about 2.5 mm ± 0.5 mm. As an example, FIG. 13a is a schematic diagram illustrating a beam waist according to some embodiments of the disclosure. As shown in FIG. 13a, when an actual beam waist diameter is below 2.5 mm, a pupil diameter is greater than the beam waist diameter. In this case, it may be ensured that, in a case that the pupil contracts, all the light enters the eye, and the spot power output by the system is all received and utilized by the fundus.

In other embodiments, the case that the power adjustment is completed by controlling the beam waist size at the light-entry position on the ocular surface is as follows. In a case that the beam waist diameter is greater than the specified threshold, the light-entry power is determined based on a power distribution of the emission power of the broad-spectrum light source and the size of the fundus spot. As an example, FIG. 13b is a schematic diagram illustrating a beam waist according to other embodiments of the disclosure. As shown in FIG. 13b, when the actual beam waist diameter is above 2.5 mm, the pupil diameter is smaller than the beam waist diameter. In a case that the spot power output by the system is in a Gaussian distribution, assuming that the beam waist diameter is greater than 2.5 mm, a proportion of the power received by the pupil of the eyes of the user is about 90% to 100%, and a degree of overlap between the pupil and a power concentration region of the output spot is relatively high. Alternatively, the spot power output by the system is in a uniform distribution, and the proportion of the power received by the pupil of the eyes of the user depends on a ratio of a pupil area to an area of the spot output by the system.

It should be further noted that, in some embodiments, an aperture parameter of the first aperture has a higher degree of influence on the power distribution of the fundus spot, and an aperture parameter of the second aperture has a higher degree of influence on the size of the fundus spot. Thus, the power of the fundus spot may be controlled mainly by adjusting the aperture size of the first aperture, and the size of the fundus spot may be controlled mainly by adjusting the aperture size of the second aperture. As an example, when performing spot shaping, the aperture of the second aperture may be adjusted first based on a required spot size, then a first spot power adjustment curve is determined based on the aperture of the second aperture, and then the aperture of the first aperture is adjusted based on the first spot power adjustment curve, so that the power of the fundus spot meets a requirement. Further, an exemplary expression of the first spot power adjustment curve is W=a1X14+ a2X13+ a3X12+ a4X1+ a5, in which W represents a spot power, X1 represents the aperture of the first aperture, and a1, a2, a3, a4 and a5 represent polynomial coefficients of the first spot power adjustment curve, and values of the coefficients may be obtained by looking up a table based on the spot size and the aperture size of the second aperture.

Further, in some embodiments, an influence of the first aperture on the spot size may be ignored, and in this case, the spot size and the aperture size of the second aperture have a one-to-one correspondence, and the correspondence may be represented by a preset spot size adjustment curve or a spot size mapping table. As an example, the preset spot size adjustment curve may be expressed as S =-0.42X2 + 3.0533, in which S represents a spot size, and X2 represents the aperture of the second aperture. An array of mapping relationships existing in the spot size mapping table is the aperture and the spot size satisfying the preset spot size adjustment curve, which is not described in detail herein.

In some embodiments, the color temperature adjuster 9 is configured to adjust a color temperature of light to be projected onto fundus of the user by transmitting light with a specified color temperature range. In some embodiments, the color temperature adjuster 9 may be a warm-light transparent plate, which may transmit light with a color temperature range of approximately 3500 K to 5000 K. By adding the color temperature adjuster, the light may be made softer, without causing harm and stimulation to the eyes.

Those skilled in the art should understand that FIG. 12 only shows an exemplary structure of the myopia illumination system. In other embodiments, the light homogenizing element 6 and the condenser lens 7 may be arranged at different positions. For example, the broad-spectrum light source 1, the light homogenizing element 6 and the condenser lens 7 are arranged in sequence, or the broad-spectrum light source 1, the condenser lens 7 and the light homogenizing element 6 are arranged in sequence, which is not limited herein. The spectrum shaping system 5 and the color temperature adjuster 9 may also be arranged at different positions, for example, the spectrum shaping system 5 may be arranged at any one of the following positions: a position between the broad-spectrum light source 1 and the condenser lens 7, a position between the broad-spectrum light source 1 and the light homogenizing element 6, a position between the light homogenizing element 6 and the condenser lens 7, a position between the condenser lens 7 and the aperture 8, a position between the light homogenizing element 6 and the aperture 8, a position between the aperture 8 and the illuminating lens 31, a position between the illuminating lens 31 and the projecting lens 32, or a position in a direction of the projecting lens 32 away from the broad-spectrum light source 1, etc. The beam shaper is similar to the spectrum shaper, and both may be arranged at any position between the broad-spectrum light source and the eyes of the user, and may be selected based on actual design needs, which is not limited herein. Similarly, the color temperature adjuster 9 may also be arranged at any position on an optical path from the broad-spectrum light source 1 to the pupil 10 of the human eye, and the color temperature adjuster 9 and the spectrum shaping system 5 may also be arranged in the myopia illumination system in any relative positional relationship. For example, light may first pass through the color temperature adjuster 9 to complete color temperature adjustment and then enter the spectrum shaping system 5, or may first pass through the spectrum shaping system 5 to undergo spectrum shaping and then enter the color temperature adjuster 9, which is not limited in embodiments of the disclosure.

Those skilled in the art should also understand that the myopia illumination system described above in conjunction with FIG. 12 is only an optional example, and in practical applications, the optical elements described in the above embodiments may be added, deleted or changed according to needs. For example, in other embodiments, the myopia illumination system may only include: the broad-spectrum light source 1, the condenser lens 7, the beam shaping system 2, the spectrum shaping system 5, the illuminating lens 31 and the projecting lens 32. For another example, in other embodiments, the myopia illumination system may only include: the broad-spectrum light source 1, the power monitor 4, the light homogenizing element 6, the beam shaping system 2, the illuminating lens 31 and the projecting lens 32. Functions of the components provided in the myopia illumination system have been described in detail in the above embodiments, which is not repeated herein.

Further, electric components in the myopia illumination system provided by any one of the above embodiments may be powered by a regulated power supply such as a battery. For example, a lithium battery may be used to supply power to the broad-spectrum light source, the power monitor, etc. Using of the regulated power supply may ensure the stability of the power of the myopia illumination system. In addition, the myopia illumination system may also monitor a circuit of the regulated power supply in real time, so that in a case that a power level is low, for example, the power level is lower than 5%, a low power level is prompted to notify the user to replace the battery in time.

Based on the myopia illumination system provided above, the disclosure may also provide a myopia illumination device. The myopia illumination device includes the myopia illumination system described in any one of the above embodiments. Further, in some embodiments, the myopia illumination device may use the myopia illumination system including the beam shaping system described in any one of the above embodiments to output an annular spot meeting requirements. An inner diameter of the annular spot output by the myopia illumination system may be adjusted based on measurement data of eyes of a user. As an example, the measurement data of the eyes of the user may be collected by an external ocular measurement device, such as a comprehensive ocular measurement instrument.

As an example, the myopia illumination device may be implemented as a smart device customized for an individual user. The measurement is performed on the eyes of the user by the comprehensive ocular measurement instrument, for example, a retinal photo of the fundus of the user is taken, so as to identify and determine tissue division of the retinal region and determine a diameter of a macular fovea of the user. Assuming that the diameter of the macular fovea of the user is 1.5 mm, when configuring the myopia illumination device, the inner diameter of the annular spot output by the myopia illumination system may be adjusted to 1.5 mm via the beam shaping system, so as to achieve the customized myopia illumination treatment.

As another example, a power of the myopia illumination device may be pre-calibrated as one or more gear positions. When using the device, the user may select an appropriate gear position for use based on specific situations. For example, 3 power gears are calibrated, in which a first gear is 0.3 mW, a second gear is 0.9 mW, and a third gear is 1.5 mW, etc. For people of different age groups and/or different myopia degrees, different usage modes may be set, for example, a younger age uses a low gear, and a high myopia degree uses a high gear, etc. In some embodiments, a single gear or a plurality of gears may be achieved by calibrating an emission power of the broad-spectrum light source. For example, a specified emission power or a plurality of preset emission powers may be pre-configured. After the myopia illumination device is turned on, the emission power of the broad-spectrum light source is directly set to the specified emission power or one of the plurality of preset emission powers.

Generally, the myopia illumination device performs myopia illumination treatment on both eyes of the user, and thus the myopia illumination device may be configured with two left and right eyepiece barrels, i.e., a left eyepiece barrel and a right eyepiece barrel, for accommodating the myopia illumination system. Further, a buffer pad made of a soft material (such as silicone, sponge, etc.) may also be fixed at an end of the left/right eyepiece barrel for attaching to the eyes of the user, so as to improve comfort when using the myopia illumination device.

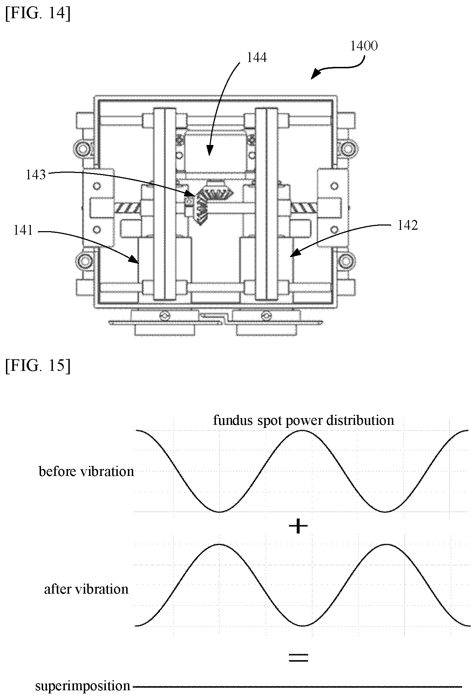

To facilitate understanding of a structure of the myopia illumination device by those skilled in the art, FIG. 14 is a schematic structural diagram illustrating a myopia illumination device 1400 according to some embodiments of the disclosure. As shown in FIG. 14, in some embodiments, a left eyepiece barrel 141 and a right eyepiece barrel 142 are connected by a spacing adjuster 143, and the spacing adjuster 143 is configured to adapt to different pupil distances by adjusting a distance between the left eyepiece barrel 141 and the right eyepiece barrel 142. The myopia illumination device, for different users, supports adjustable gear positions and also supports an adjustable pupil distance. Further, the pupil distance may also be set with different gear positions. For example, the spacing adjuster allows a spacing range of 42 mm to 76 mm, and a pupil distance gear position may be set at an interval of 2 mm, 1 mm, or 0.5 mm, etc., and the interval of the pupil distance gear positions is not limited herein.

In other embodiments, a monocular or binocular 3D camera device may also be provided on the left eyepiece barrel and/or the right eyepiece barrel, and the 3D camera device is configured to collect pupil information of the user, for example, a pupil size of the user. The pupil information may assist in adjusting a diameter of the convergent light beam entering the eyes or adjusting a gear position power of the myopia illumination device, so as to ensure the effectiveness of light entering the eyes.

Further, the myopia illumination device may also include a mechanical adjuster, which is configured to adjust an axis of an optical path of the myopia illumination system in the eyepiece barrel. In combination with the 3D camera device provided on the left eyepiece barrel and/or the right eyepiece barrel, the axis direction of the optical path of the myopia illumination system may be changed to align a center of the annular spot output by the myopia illumination system with a gaze direction of the human eye, so as to ensure the efficiency of the spot entering the eyes.

In addition to adjusting the axis of the optical path of the myopia illumination system in the eyepiece barrel, in some embodiments, the mechanical adjuster may further adjust a position of the visual target pattern. The position of the visual target pattern is adjusted to guide and change the gaze direction of the user, thus avoiding visual fatigue caused by the eyes of the user staring at the same direction for a long time. It should be noted that, in the process of adjusting the position of the visual target pattern, the axis direction of the optical path of the myopia illumination system needs to be synchronously controlled, so as to ensure that the visual target pattern is always located at the center of the annular spot, thus ensuring that the annular spot is projected onto the specified area of the eyes of the user.

In order to improve the accuracy of the above adjustment process, the 3D camera device may be used to synchronously monitor a real-time movement state of the user, thus providing reference information for the adjustment of the mechanical adjuster, so as to ensure the effectiveness of the irradiation.

In addition, in order to ensure that the power distribution of the fundus spot is more uniform, in some embodiments, a vibration motor 144 may also be provided on a housing of the myopia illumination device. The vibration motor 144 is activated in a case that the myopia illumination system operates. In order to more intuitively show the light homogenizing effect of the vibration motor, FIG. 15 is a schematic diagram illustrating a comparison between power distributions before and after operation of a vibration motor according to some embodiments of the disclosure. It may be seen from FIG. 15 that, after the vibration motor vibrates at a certain frequency, waveforms of the power distributions before and after the vibration are superimposed, the fluctuation amplitude is reduced, and the power distribution is more uniform and stable. It should be further noted that a vibration direction of the vibration motor may be an arrangement direction of the left eyepiece barrel and the right eyepiece barrel, and during the vibration, one or both of the left eyepiece barrel and the right eyepiece barrel may be selected to vibrate.

In order to achieve intelligent control of the myopia illumination device, some embodiments of the disclosure further add a controller in the myopia illumination device. It should be noted that the number of the controller may be one or more. In the disclosure, the same controller may perform different functions as described below, or a plurality of different controllers may respectively perform different functions.

As an example, the power controller is connected to the broad-spectrum light source, and is configured to control the emission power of the broad-spectrum light source. For example, the emission power of the broad-spectrum light source is adjusted to a specified emission power or one of a plurality of preset emission powers based on a gear position instruction fed back by the user, thus achieving gear position adjustment of the power.

Further, the power controller may also be communicatively connected to an external ocular measurement device, and automatically adjust the gear position of the device power after receiving measurement data of the ocular measurement device. As an example, the user may perform an ocular measurement every certain period, for example, one month or half a month, to measure whether the fundus is damaged or whether a myopia diopter is improved, etc. The controller may also update the specified emission power or the preset emission power based on the information, thus updating and upgrading the preset power gear position.

The spacing controller may adjust a pupil distance gear position. As another example, the spacing controller may be connected to the spacing adjuster, and is configured to control the spacing adjuster. For a first-time user, a motion mode may be achieved by using the spacing controller and the spacing adjuster. In the motion mode, a spacing between the left/right eyepiece barrels moves cyclically between 42 mm and 76 mm, which may effectively improve the adaptability of the first-time user to red light. It should be noted that the above spacing range is only an example.

As another example, the shaping controller may also be connected to the 3D camera device to obtain the pupil information of the user collected by the 3D camera device. In some embodiments, the shaping controller is connected to the beam shaping system, so that the beam shaping system is controlled based on a pupil size of the user to adjust the diameter of the convergent light beam entering the eyes and thus form annular light matching the pupil size of the user. In other embodiments, the optical axis controller may be connected to the mechanical adjuster, so that the mechanical adjuster is controlled based on the pupil position of the user to adjust the axis of the optical path of the myopia illumination system and align with the pupil center, so as to ensure the efficiency of light from the light source entering the eyes.

It should be noted that the power controller, the spacing controller, the shaping controller, and the illumination controller may be the same controller, or may be a plurality of controllers respectively performing different functions in the myopia illumination device. The naming of the controllers is only for distinguishing different functions achieved by the controllers, which does not limit the number of the controllers.

The myopia illumination device may also internally store usage information of the device, which may include: usage requirement information and device usage information, etc. The usage requirement information may include a daily usage frequency, a usage interval duration, a usage duration, etc. For example, the usage requirement information may specify that the myopia illumination device is used twice a day, with an interval of 4 hours or more each time, and each usage lasts 3 minutes. The controller may monitor whether the device usage meets the usage requirement information, and if the usage requirement information is met, control the device to start normally; otherwise, the device cannot be used normally. For medical scenarios, the daily usage frequency may also be set to unlimited times, and a plurality of usage duration gears may be set for selection. The device usage information is used to feed back a usage condition of the device, for example, the device usage information may include a usage frequency of the device, a positioning of the device, a type of network used, a usage time, whether the device is in a normal state, a usage power, etc.

As an example, the device data such as the device usage information may be managed in a unified manner by a device management platform, and the myopia illumination device performs data interaction with the device management platform via wireless communication. The device management platform may remotely upgrade the myopia illumination system by monitoring such data. In some embodiments, the myopia illumination device may be configured with a remote upgrade module, which is controlled by the device management platform and is responsible for performing remote upgrades according to ocular measurement data of the user each time.

Based on the functions of one or more controllers described in the above embodiments, some embodiments of the disclosure may provide a control method for a myopia illumination device, and the control method is applicable to the myopia illumination device described in any one of the above embodiments. For convenience of description, the control method for a myopia illumination device is described below by taking a single controller as an example. It should be noted that, in a case that the myopia illumination device adopts a plurality of controllers, the following control methods may match and correspond to controllers of different functions, which is not repeated herein.

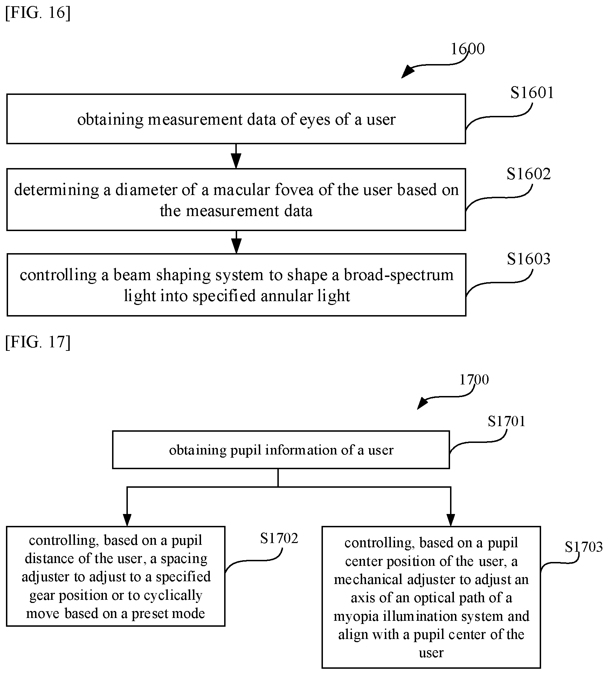

In the embodiment, the control method is performed by a controller, for example, a shaping controller. FIG. 16 is an exemplary flowchart illustrating a control method 1600 for a myopia illumination device according to some embodiments of the disclosure.

As shown in FIG. 16, in step S1601, measurement data of eyes of a user is obtained. In some embodiments, the measurement data may be collected by an external ocular measurement device such as a comprehensive ocular measurement instrument. Further, the measurement data may be collected at predetermined intervals, thus forming a data set of measurement data of a plurality of periods. The measurement data includes, but is not limited to: a diameter of a macular fovea of the user, myopia diopters of both eyes, a pupil distance of the user, an axial length, etc.

At step S1602, a diameter of a macular fovea of the user is determined based on the measurement data. The diameter of the macular fovea of the user may reflect a position and a size of a sensitive area of the eyes of the user, thus guiding a shape of an annular spot.

At step S1603, the beam shaping system is controlled to shape the broad-spectrum light into specified annular light. In this step, the controller controls a light shaping process by controlling the beam shaping system, thus forming the specified annular light, in which the implementation of the shaping function of the beam shaping system is described in detail in the above embodiments, which is not repeated herein.

In the embodiment, an inner diameter of the specified annular light is consistent with the diameter of the macular fovea of the user, thus ensuring that the macular fovea of the user may be protected from strong light stimulation.

In addition, in some embodiments, the controller may also control an emission power of the broad-spectrum light source. As an example, a power of the myopia illumination system may be preset as one or more power gear positions. When the myopia illumination device is turned on, the controller may control the emission power of the broad-spectrum light source to be a specified emission power, so that the power of the myopia illumination system is implemented as a preset power gear position, or the controller may also control the emission power of the broad-spectrum light source to be one of the plurality of preset emission powers, so that the power of the myopia illumination system is implemented as one of preset power gear positions.

Further, when the measurement data includes measurement data obtained in a plurality of periods, the controller may also determine, based on the measurement data, information such as whether there is damage to the fundus of the user, a degree of damage, or whether a myopia diopter is improved, etc., thus updating the specified emission power or the preset emission power, so as to achieve adjustment of the power gear position.

In addition to adjusting the power gear position, in other embodiments, a controller, for example, a spacing controller, may control the myopia illumination device to adapt to different pupil distances. In the embodiment, the myopia illumination device further includes: a left eyepiece barrel, a right eyepiece barrel, and a spacing adjuster provided between the left eyepiece barrel and the right eyepiece barrel, in which the spacing adjuster is configured to adjust a distance/spacing between the left eyepiece barrel and the right eyepiece barrel.

FIG. 17 is an exemplary flowchart illustrating a control method 1700 for a myopia illumination device according to other embodiments of the disclosure. A controller, for example, a spacing controller, may also execute the control method as shown in FIG. 17.

At step S1701, pupil information of a user is obtained. In the embodiment, the pupil information includes a pupil distance of the user. In some embodiments, the pupil information may be collected by a 3D camera device provided on an eyepiece barrel. The 3D camera device may be a monocular or binocular camera device. In addition, the 3D camera device may be provided on either or both of a left eyepiece barrel and a right eyepiece barrel. The 3D camera device is arranged at a position adjacent to an eye placement location of the eyepiece barrel, so as to facilitate the 3D camera device to collect the pupil information of the user, and the collected pupil information is sent to the controller for subsequent operations such as adjusting a pupil distance gear position.

At step S1702, based on the pupil distance of the user, the spacing adjuster is controlled to adjust to a specified gear position or to cyclically move based on a preset mode. In some embodiments, the controller may control the spacing adjuster to adjust the spacing between the left eyepiece barrel and the right eyepiece barrel to be consistent with the pupil distance of the user, in which the spacing refers to a spacing/distance between axes of the left eyepiece barrel and the right eyepiece barrel. In other embodiments, the controller may control the spacing adjuster to adjust to the specified gear position, and the specified gear position may be one of a plurality of preset spacing gear positions. For example, a spacing range of the spacing adjuster is 42 mm to 76 mm, and a spacing gear position may be generated at intervals of 2 mm, 1 mm, or 0.5 mm. In other embodiments, the myopia illumination device may also be provided with a motion mode as the preset mode, and in the motion mode, the spacing adjuster may cyclically move within the spacing range of the spacing adjuster, so as to improve the adaptability of the first-time user to red light.

In addition to the pupil distance, a controller, for example, an optical axis controller, may also control an eye-entry angle of the annular light. As an example, the myopia illumination device may further include a mechanical adjuster for adjusting the axis of the optical path of the myopia illumination system. After the pupil information of the user is obtained in step S1701, the controller may further execute step S1703. In step S1703, based on a pupil center position of the user, the mechanical adjuster is controlled to adjust the axis of the optical path of the myopia illumination system and align with a pupil center of the user.

Further, in some embodiments, the myopia illumination device further includes a visual target system, configured to generate light of a visual target pattern. After the pupil information of the user is obtained in step S1701, the controller may further control, based on the pupil information of the user, the mechanical adjuster to adjust a direction of the light of the visual target pattern and align with the pupil center of the user.

As an example, the visual target system includes a visual target light source and a beam splitter. The relative positions of the visual target light source and the beam splitter in the myopia illumination device are described in detail in the above embodiments, which is not repeated herein. In the example, the controller may complete adjustment of the direction of the light of the visual target pattern by controlling the mechanical adjuster to adjust one or more of: a position of the visual target light source, a position of the beam splitter, or an angle of the beam splitter.

In addition, some embodiments of the disclosure may also obtain a pupil size of the user via the 3D camera device provided on the myopia illumination device, for reference by the controller, and controlling the beam shaping system to adjust a beam waist diameter of the annular light entering the eyes to adapt to the pupil size. Further, the 3D camera device may calculate and capture the pupil size in real time, and the controller may also control a beam shaper in real time based on a result collected by the 3D camera device in real time.

In some embodiments of the disclosure, the myopia illumination systems in the left eyepiece barrel and the right eyepiece barrel may be individually controlled. As an example, after the controller obtains measurement data of the eyes of the user, the controller may, based on myopia diopters and/or axial length information of the eyes of the user in the measurement data, control the myopia illumination system in the left eyepiece barrel to be turned on in a case that the user has myopia only in the left eye, or control the myopia illumination system in the right eyepiece barrel to be turned on in a case that the user has myopia only in the right eye, or control both of the myopia illumination systems to be turned on in a case that the user has myopia in both eyes. Further, in other embodiments, the controller may further control a turn-on duration of the myopia illumination system in the left eyepiece barrel and a turn-on duration of the myopia illumination system in the right eyepiece barrel, thus achieving time-division management. For example, for a user with different myopia degrees in the two eyes, an irradiation time for the eye with a high myopia degree is 3 min, and an irradiation time for the eye with a low myopia degree is 2 min or 1 min, and the time may be in units of min or second, which is not limited herein.

Further, in other embodiments, the controller may further control an emission power of the myopia illumination system in the left eyepiece barrel and an emission power of the myopia illumination system in the right eyepiece barrel. For example, for the user with different myopia degrees in the two eyes, a higher emission power is used on the side of the eye with the high myopia degree, and a lower emission power is used on the side of the eye with the low myopia degree.

The terms "first" or "second" used in the disclosure to refer to numbers or ordinals are only for descriptive purposes, and cannot be construed as explicitly or implicitly indicating relative importance or implicitly indicating the number of the indicated technical features. Thus, a feature defined with "first" or "second" may explicitly or implicitly include at least one such feature. In the description of the disclosure, "a plurality of" means at least two, for example, two, three or more, unless otherwise explicitly and specifically defined.

Although a plurality of embodiments of the disclosure are shown and described herein, it is obvious to those skilled in the art that such implementations are provided only by way of example. Those skilled in the art may think of many modifications, changes and alternatives without departing from the idea and spirit of the disclosure. It should be understood that alternatives to the embodiments of the disclosure described herein may be adopted in the process of practicing the disclosure. The appended claims are intended to define the protection scope of the disclosure, and thus cover equivalents or alternatives within the scope of these claims.

## Claims

1. A myopia illumination system, comprising: a broad-spectrum light source, configured to emit broad-spectrum light; and
a projection system, configured to project the broad-spectrum light onto eyes of a user.

2. The system according to claim 1, further comprising: a beam shaping system, configured to shape the broad-spectrum light to form annular light; and the projection system is configured to project the annular light onto the eyes of the user to form an annular spot.

3. The system according to claim 2, wherein the beam shaping system comprises: a substrate, the substrate comprising an annular transparent portion and an opaque portion located at a center of the annular transparent portion; or
the beam shaping system comprises: a spectrum shaper, the spectrum shaper comprising an annular transparent portion and an opaque portion located at a center of the annular transparent portion.

4. The system according to claim 2, further comprising: a visual target system, configured to generate light of a visual target pattern, wherein the light generated is projected onto the eyes of the user together with the annular light.

5. The system according to claim 4, wherein the visual target system comprises: a visual target transparent portion arranged in a spectrum shaper of the beam shaping system and located at a center of an opaque portion of the spectrum shaper, and
the visual target transparent portion is configured to form the annular light with the visual target pattern at the center by transmitting a portion of the broad-spectrum light.

6. The system according to claim 4, wherein the visual target system comprises: a visual target light source and a beam splitter, an output optical path of the visual target light source coinciding with a first optical path of the beam splitter, and a second optical path of the beam splitter coinciding with a propagation optical path of the annular light;
wherein the first optical path is one of a reflection optical path or a projection optical path, the second optical path is the other of the reflection optical path or the projection optical path different from the first optical path, and a position where a visual target light output by the visual target light source is projected onto the eyes of the user is located at a center of the annular spot.

7. The system according to any one of claims 3 to 6, comprising at least one of the following features (1) to (3):
(1) a material of the annular transparent portion comprises any one of: glass, crystal, plastic, or resin;
(2) a material of the opaque portion is an opaque material; or
(3) the opaque portion is made of the same material as the annular transparent portion but is subjected to an opaque treatment.

8. The system according to claim 7, wherein the opaque portion comprises:
black acrylic; or
the opaque treatment comprises: sandblasting, painting, or attaching a black-dot annular transparent film.

9. The system according to any one of claims 2 to 8, wherein a size of the annular spot satisfies any one of following situations:
an annular width k of the annular spot is 0.0038 mm to 3 mm, and a distance r1 from a spot center to an annular centerline is 0.0038 mm to 3 mm;
an inner diameter of the annular spot is between 0.0076 mm and 6 mm;
an inner diameter d1 of the annular spot is 1.5 mm, and an outer diameter d2 of the annular spot is 2.5 mm;
the inner diameter d1 of the annular spot is 2.5 mm, and the outer diameter d2 of the annular spot is 5.5 mm; or
the inner diameter d1 of the annular spot is 1.5 mm, and the outer diameter d2 of the annular spot is 5.5 mm.

10. The system according to any one of claims 1 to 9, wherein a spectral range of the broad-spectrum light source is a spectral range covering any spectral cutoff width within a range of 300 nm to 700 nm.

11. The system according to any one of claims 1 to 10, wherein the broad-spectrum light source comprises at least one of a light-emitting diode (LED) lamp, an incandescent lamp, or a fluorescent lamp.

12. The system according to any one of claims 1 to 11, further comprising:
a spectrum shaping system, configured to form light with a specified spectrum by shaping a spectrum of the broad-spectrum light; and
the projection system is configured to project the light with the specified spectrum onto the eyes of the user.

13. The system according to claim 12, wherein the spectrum shaping system comprises one or more spectrum shapers, and a spectral range of one or more of the spectrum shapers satisfies any one of following situations:
a spectral cutoff width is 5 nm to 60 nm, and a central wavelength is selected from any wavelength within a wavelength range of 360 nm to 400 nm or within a wavelength range of 640 nm to 700 nm;
a low-pass cutoff range is approximately 640 nm to 660 nm, and a high-pass cutoff range is approximately 670 nm to 700 nm; or
a low-pass cutoff range is approximately 360 nm to 375 nm, and a high-pass cutoff range is approximately 385 nm to 400 nm.

14. The system according to claim 13, wherein the spectrum shaper comprises any one or more or a combination of: a filter; a transmission grating filter; a reflection grating filter; a prism; a lens; a liquid crystal spectrum transmission plate; or a spectrum absorber.

15. The system according to claim 13, wherein a spectral characteristic of the spectrum shaper comprises any one of: high-pass; low-pass; band-pass; or multi-band-pass.

16. The system according to any one of claims 1 to 15, further comprising:
a color temperature adjuster, configured to adjust a color temperature of light to be projected onto fundus of the user by transmitting light with a specified color temperature range.

17. The system according to claim 16, wherein the color temperature adjuster is a warm-light transparent plate, and the specified color temperature range is approximately 3500 K to 5000 K.

18. The system according to any one of claims 1 to 17, further comprising a light homogenizing plate, wherein a smooth surface of the light homogenizing plate faces the broad-spectrum light source, and a rough surface of the light homogenizing plate faces the eyes of the user.

19. The system according to claim 18, wherein a distance between the light plate and the broad-spectrum light source is 0 mm to 1 mm.

20. The system according to any one of claims 18 to 19, further comprising
a power monitor, configured to control an emission power of the broad-spectrum light source within a preset range by monitoring the emission power.

21. The system according to claim 20, wherein the power monitor implements the monitoring by receiving light that is emitted from the broad-spectrum light source and reflected by the smooth surface of the light homogenizing plate.

22. The system according to claim 20, wherein the preset range is 0.1 mW to 1 W.

23. The system according to any one of claims 1 to 22, wherein the projection system comprises a first aperture, an illuminating lens, a second aperture and a projecting lens arranged in sequence along an optical path, the second aperture being in close contact with the illuminating lens,
wherein the first aperture is optically conjugate with a light-entry position on an ocular surface of the eyes of the user;
the second aperture is optically conjugate with a fundus spot of the eyes of the user; and
a light-entry power of the myopia illumination system is determined based on one or more of: a beam waist size at the light-entry position, a size of the fundus spot, or an emission power of the broad-spectrum light source.

24. The system according to claim 23, wherein:
in a case that a beam waist diameter does not exceed a specified threshold, the light-entry power is equal to the emission power of the broad-spectrum light source; or
in a case that the beam waist diameter is greater than the specified threshold, the light-entry power is determined based on a power distribution of the emission power of the broad-spectrum light source and the size of the fundus spot.

25. The system according to claim 24, wherein the specified threshold is 2.5 mm ± 0.5 mm.

26. A myopia illumination device, comprising: the myopia illumination system according to any one of claims 1 to 25.

27. The device according to claim 26, wherein an inner diameter of an annular spot output by the myopia illumination system is adjusted based on measurement data of eyes of a user.

28. The device according to any one of claims 26 to 27, further comprising: a power controller,
wherein the power controller is communicatively connected to an external ocular measurement device, and is configured to obtain the measurement data of the eyes of the user, and control, based on the measurement data, an emission power of a broad-spectrum light source to be a specified emission power or one of a plurality of preset emission powers, and update the specified emission power or the preset emission power.

29. The device according to any one of claims 26 to 28, further comprising:
a left eyepiece barrel and a right eyepiece barrel, wherein the left eyepiece barrel and the right eyepiece barrel each are provided with the myopia illumination system, and the left eyepiece barrel and the right eyepiece barrel are connected by a spacing adjuster, and the spacing adjuster is configured to adapt to different pupil distances by adjusting a distance between the left eyepiece barrel and the right eyepiece barrel.

30. The device according to claim 29, further comprising: a spacing controller, wherein the spacing controller is connected to the spacing adjuster and is configured to control the spacing adjuster to adjust to a specified gear position or to cyclically move based on a preset mode.

31. The device according to any one of claims 29 to 30, wherein a 3D camera device is provided on the left eyepiece barrel and/or the right eyepiece barrel, configured to collect pupil information of the user.

32. The device according to claim 31, further comprising: an optical axis controller and a mechanical adjuster;
wherein the optical axis controller is respectively connected to the mechanical adjuster and the 3D camera device, and is configured to control, based on the pupil information, the mechanical adjuster to adjust an axis of an optical path of the myopia illumination system.

33. The device according to any one of claims 31 to 32, further comprising: a shaping controller;
wherein the shaping controller is respectively connected to the 3D camera device and the beam shaping system, and is configured to control, based on the pupil information, the beam shaping system to form a beam waist diameter of the annular light entering the eyes that matches a pupil size.

34. The device according to any one of claims 26 to 33, wherein device data of the myopia illumination device is managed in a unified manner by a device management platform, and the myopia illumination device performs data interaction with the device management platform via wireless communication.

35. The device according to any one of claims 26 to 34, wherein a vibration motor is provided on a housing of the myopia illumination device, and the vibration motor is activated in a case that the myopia illumination system operates.

36. A control method for a myopia illumination device, wherein the myopia illumination device comprises: a broad-spectrum light source, a beam shaping system, a projection system and a shaping controller; a broad-spectrum light emitted by the broad-spectrum light source is shaped by the beam shaping system, and annular light formed by shaping is projected by the projection system onto eyes of a user to form an annular spot;
wherein the control method performed by the shaping controller comprises:
obtaining measurement data of the eyes of the user;
determining a diameter of a macular fovea of the user based on the measurement data; and
controlling the beam shaping system to shape the broad-spectrum light into specified annular light, wherein an inner diameter of the specified annular light is consistent with the diameter of the macular fovea of the user.

37. The method according to claim 36, wherein the myopia illumination device further comprises: a power controller, and after obtaining the measurement data of the eyes of the user, the control method performed by the power controller comprises:
controlling, based on the measurement data, an emission power of the broad-spectrum light source to be a specified emission power or one of a plurality of preset emission powers; and/or,
updating the specified emission power or the preset emission power based on the measurement data, wherein the measurement data comprises measurement data obtained in a plurality of periods.

38. The method according to claim 36, wherein the myopia illumination device further comprises: a left eyepiece barrel, a right eyepiece barrel, a spacing adjuster provided between the left eyepiece barrel and the right eyepiece barrel, and a spacing controller;
the control method performed by the spacing controller comprises:
controlling, based on a pupil distance of the user, the spacing adjuster to adjust to a specified gear position or to cyclically move based on a preset mode.

39. The method according to claim 36, wherein the myopia illumination device further comprises: a mechanical adjuster for adjusting an axis of an optical path of the myopia illumination system, and an optical axis controller;
wherein the control method performed by the optical axis controller comprises:
controlling, based on pupil information of the user, the mechanical adjuster to adjust the axis of the optical path of the myopia illumination system and align with a pupil center of the user.

40. The method according to claim 39, wherein the myopia illumination device further comprises: a visual target system, configured to generate light of a visual target pattern, wherein the light generated is projected onto the eyes of the user together with the annular light;
wherein the control method performed by the optical axis controller comprises:
controlling, based on the pupil information of the user, the mechanical adjuster to adjust a direction of the light of the visual target pattern and align with the pupil center of the user.

41. The method according to claim 40, wherein the visual target system comprises: a visual target light source and a beam splitter, an output optical path of the visual target light source coinciding with a first optical path of the beam splitter, and a second optical path of the beam splitter coinciding with a propagation optical path of the annular light;
wherein the control method performed by the optical axis controller further comprises:
controlling, based on the pupil information of the user, the mechanical adjuster to adjust one or more of: a position of the visual target light source, a position of the beam splitter, or an angle of the beam splitter.

42. The method according to claim 36, wherein the myopia illumination device further comprises: a left eyepiece barrel and a right eyepiece barrel, the left eyepiece barrel and the right eyepiece barrel each being provided with a myopia illumination system; and
after obtaining the measurement data of the eyes of the user, the control method further comprises:
controlling, based on the measurement data, one or both of the myopia illumination systems in the left eyepiece barrel and the right eyepiece barrel to be turned on; and/or,
controlling, based on the measurement data, a turn-on duration of the myopia illumination system in the left eyepiece barrel and a turn-on duration of the myopia illumination system in the right eyepiece barrel; and/or,
controlling, based on the measurement data, an emission power of the myopia illumination system in the left eyepiece barrel and an emission power of the myopia illumination system in the right eyepiece barrel;
wherein the measurement data comprises myopia diopters and/or axial length information of the eyes of the user.
